## Europäisches Patentamt

## European Patent Office

## Office européen des brevets

(19)

(11) Veröffentlichungsnummer: **0 468 539 B1**

(12) **EUROPÄISCHE PATENTSCHRIFT**

(45) Veröffentlichungstag der Patentschrift: **13.09.95**

(51) Int. Cl.⁶: **C12N 15/15**

(21) Anmeldenummer: **91114411.1**

(22) Anmeldetag: **21.11.86**

(60) Veröffentlichungsnummer der früheren Anmeldung nach Art. 76 EPÜ: **0 227 938**

(54) **Hirudinderivate.**

(30) Priorität: **27.11.85 DE 3541856**

(43) Veröffentlichungstag der Anmeldung:
**29.01.92 Patentblatt 92/05**

(45) Bekanntmachung des Hinweises auf die Patenterteilung:
**13.09.95 Patentblatt 95/37**

(84) Benannte Vertragsstaaten:
**AT BE CH DE ES FR GB GR IT LI LU NL SE**

(56) Entgegenhaltungen:
**EP-A- 158 564**
**EP-A- 171 024**
**EP-A- 211 299**

(73) Patentinhaber: **HOECHST AKTIENGESELL-SCHAFT**

**D-65926 Frankfurt am Main (DE)**

(72) Erfinder: **Habermann, Paul, Dr.**
**Heimchenweg 80**
**W-6230 Frankfurt am Main 80 (DE)**
Erfinder: **Wengenmayer, Friedrich, Dr.**
**Am Seyenbach 38**
**W-6238 Hofheim a. Ts. (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Bei der gentechnischen Herstellung eukaryotischer Proteine wird in Bakterien häufig nur eine geringe Ausbeute erhalten, insbesondere bei kleinen Proteinen mit einem Molgewicht bis zu etwa 15 000 Dalton, deren Strukturen Disulfidbrücken enthalten. Man nimmt an, daß die gebildeten Proteine durch wirtseigene Proteasen rasch abgebaut werden. Man konstruiert deshalb zweckmäßig Genstrukturen, die für Fusionsproteine codieren, wobei der unerwünschte Anteil des Fusionsproteins ein wirtseigenes Protein ist, das nach der Isolation der Primärproduktes nach an sich bekannten Methoden abgespalten wird.

Es wurde nun überraschenderweise gefunden, daß ein N-terminaler Anteil von Interleukin-2, der im wesentlichen den ersten 100 Aminosäuren entspricht, besonders gut zur Herstellung von Fusionsproteinen geeignet ist. Man erhält also als Primärprodukt ein Fusionsprotein, das völlig oder zum ganz überwiegenden Teil aus eukaryotischen Proteinsequenzen besteht. Überraschenderweise wird dieses Protein offenbar in dem betreffenden Wirtsorganismus nicht als Fremdprotein erkannt und nicht sofort wieder abgebaut. Ein weiterer Vorteil ist, daß die erfindungsgemäßen Fusionsproteine schwer löslich bis unlöslich sind und sich somit einfach, zweckmäßig durch Zentrifugation, von den löslichen Proteinen abtrennen lassen.

Da es erfindungsgemäß hinsichtlich der Funktion als "Ballast-Anteil" des Fusionsproteins nicht darauf ankommt, daß der Interleukin-2-Anteil ein biologisch aktives Molekül darstellt, kommt es insofern auch nicht auf die exakte Struktur des Interleukin-2-Anteils an. Es genügt hierfür, daß im wesentlichen die ersten 100 N-terminalen Aminosäuren vorliegen. Es ist also beispielsweise möglich, am N-Terminus Variationen vorzunehmen, die eine Spaltung des Fusionsproteins erlauben, falls das erwünschte Protein N-terminal dazu angeordnet ist. Umgekehrt kann man C-terminal Variationen vornehmen, um die Abspaltung des gewünschten Proteins zu ermöglichen oder zu erleichtern, falls dieses im Fusionsprotein - wie üblich - C-terminal gebunden ist.

Die für Human-Interleukin-2, im folgenden "IL-2", codierende natürliche DNA-Sequenz ist aus der europäischen Patentanmeldung mit der Veröffentlichungsnummer EP-A1-0 091 539 bekannt. Die dort aufgeführte Literatur bezieht sich auch auf Mäuse- und Ratten-IL-2. Diese Säuger-DNA kann zur Synthese der erfindungsgemäßen Proteine herangezogen werden. Zweckmäßiger geht man jedoch von einer synthetischen DNA aus, besonders vorteilhaft von der DNA für Human-IL-2, die in der (nicht vorveröffentlichten) deutschen Offenlegungsschrift 34 19 995 (entsprechend der unter der Nummer 0 163 249 veröffentlichten europäischen Patentanmeldung) vorgeschlagen wurde. Diese synthetische DNA-Sequenz ist im Anhang wiedergegeben (DNA-Sequenz I). Diese synthetische DNA hat nicht nur den Vorzug, daß sie in der Codon-Wahl auf die Gegebenheiten des am häufigsten verwendeten Wirts, E. coli, abgestimmt ist, sondern sie enthält auch eine Reihe von Schnittstellen für Restriktionsendonucleasen, von denen erfindungsgemäß Gebrauch gemacht werden kann. In der folgenden Tabelle 1 ist eine Auswahl der geeigneten Schnittstellen am Anfang bzw. in der Region des 100. Tripletts wiedergegeben. Hierdurch ist jedoch nicht ausgeschlossen, daß in dem dazwischenliegenden Bereich Variationen in der DNA vorgenommen werden, wobei von den in der vorstehend genannten Patentanmeldung aufgeführten weiteren Schnittstellen Gebrauch gemacht werden kann.

Tabelle 1

| Restriktionsenzym | Erkennungssequenz | | Position des ersten Nucleotids der Erkennungssequenz (codierender Strang) |
|---|---|---|---|
| Aha II, Ban I, | 5'          3' | | |
| Hae II, Nar I, | GGCGCC | | 8 |
| Ban II, Sac I, Sst I | GAGCTC | | 291 |
| Hha I | GCGC | | 9 |
| Hinf I | GACTC | | 35 |
| Pvu I | CGATCG | | 346 |
| Taq I | TCGA | | 387 |

Wird von den Nucleasen Ban II, Sac I oder Sst I Gebrauch gemacht, so erhält man eine IL-2-Teilsequenz, die für etwa 95 Aminosäuren codiert. Diese Länge ist im allgemeinen ausreichend, um ein unlösliches Fusionsprotein zu erhalten. Wenn die Schwerlöslichkeit, beispielsweise bei einem gewünschten hydrophilen eukaryotischen Protein, noch nicht ausreicht, man aber - um so wenig "Ballast" wie möglich zu produzieren - nicht von den näher am C-Terminus liegenden Schnittstellen Gebrauch machen will, so kann man durch entsprechende Adapter bzw. Linker die DNA-Sequenz am N- und/oder C-terminalen Ende verlängern und so den "Ballast"-Anteil "maßschneidern". Man kann natürlich auch die DNA-Sequenz - mehr oder weniger - bis zum Ende nutzen und so - gegebenenfalls modifiziertes - biologisch aktives IL-2 als "Nebenprodukt" erzeugen bzw. ein bifunktionelles Protein erzeugen, das IL-2 Wirkung zusätzlich zur Wirkung des codierten Proteins zeigt.

Es werden somit Fusionsproteine der allgemeinen Formel eingesetzt

Met - X - Y - Z      (Ia)

oder

Met - Z - Y - X      (Ib)

in der X im wesentlichen die Aminosäurefolge der etwa 100 ersten Aminosäuren von vorzugsweise menschlichem IL-2 bedeutet, Y eine direkte Bindung bedeutet, falls die zum gewünschten Protein benachbarte Aminosäure oder Aminosäurenfolge eine Abspaltung des gewünschten Proteins ermöglicht, oder andernfalls ein Brückenglied aus einer oder mehreren genetisch codierbaren Aminosäuren, das die Abspaltung ermöglicht, und Z eine Sequenz aus genetisch codierbaren Aminosäuren ist, die für das gewünschte Protein codiert.

Wie sich aus den Formeln Ia und Ib ergibt - und wie es auch schon vorstehend erwähnt wurde - ist es möglich, das gewünschte Protein vor oder nach dem IL-2-Anteil zur Expression zu bringen. Zur Vereinfachung wird im folgenden im wesentlichen die erste Möglichkeit erläutert, die der herkömmlichen Methode zur Herstellung von Fusionsproteinen entspricht. Wenn also im folgenden diese "klassische" Variante beschrieben wird, soll die andere Alternative hierdurch nicht ausgeschlossen werden.

Die Spaltung des Fusionsproteins kann in an sich bekannter Weise chemisch oder enzymatisch erfolgen. Die Wahl der geeigneten Methode richtet sich vor allem nach der Aminosäuresequenz des gewünschten Proteins. Wenn dieses beispielsweise kein Methionin enthält, kann Y Met bedeuten, worauf eine chemische Spaltung mit Chlor- oder Bromcyan erfolgt. Steht im Bindeglied Y am Carboxyterminus Cystein oder steht Y für Cys, so kann eine enzymatische Cystein-spezifische Spaltung oder eine chemische Spaltung, beispielsweise nach spezifischer S-Cyanylierung, folgen. Steht im Brückenglied Y am Carboxyterminus Tryptophan oder Y für Trp, so kann eine chemische Spaltung mit N-Brom-succinimid erfolgen.

Proteine, die in ihrer Aminosäuresequenz nicht

Asp - Pro

enthalten und hinreichend säurestabil sein, können in an sich bekannter Weise proteolytisch gespalten werden. Hierdurch erhält man Proteine, die N-terminal Prolin bzw. C-terminal Asparaginsäure enthalten. Auf diese Weise können also auch modifizierte Proteine synthetisiert werden.

Die Asp-Pro-Bindung kann noch säurelabiler gestaltet werden, wenn dieses Brückenglied $(Asp)_n$-Pro bzw. Glu-$(Asp)_n$-Pro ist, wobei n 1 bis 3 bedeutet.

Das Fusionsprotein wird durch Expression in einem geeigneten Expressionssystem in an sich bekannter Weise gewonnen. Hierfür eignen sich alle bekannten Wirts-Vektor-Systeme, also beispielsweise Säugerzellen und Mikroorganismen, beispielsweise Hefen und vorzugsweise Bakterien, insbesondere E. coli.

Die DNA-Sequenz, die für das gewünschte Protein codiert, wird in bekannter Weise in einen Vektor eingebaut, der in dem gewählten Expressionssystem eine gute Expression gewährleistet.

In bakteriellen Wirten wählt man zweckmäßig den Promotor und Operator aus der Gruppe lac, tac, trp, $P_L$ oder $P_R$ des Phagen $\lambda$, hsp, omp oder einen synthetischen Promotor, wie sie beispielsweise in der deutschen Offenlegungsschrift 34 30 683 (Europäische Patentanmeldung mit der Veröffentlichungsnummer 0 173 149) vorgeschlagen sind. Vorteilhaft ist die tac Promotor-Operator-Sequenz, die inzwischen handelsüblich ist (z.B. Expressionsvektor pKK223-3, Pharmacia, "Molecular Biologicals, Chemicals and Equipment for Molecular Biology", 1984, S. 63).

Bei der Expression des erfindungsgemäßen Fusionsproteins kann es sich als zweckmäßig erweisen, einzelne Tripletts der ersten Aminosäuren nach dem ATG-Start-Codon zu verändern, um eine eventuelle Basenpaarung auf der Ebene der mRNA zu verhindern. Solche Veränderungen, ebenso wie Veränderungen, Deletionen oder Additionen einzelner Aminosäuren im IL-2-Proteinanteil, sind dem Fachmann geläufig und ebenfalls Gegenstand der Erfindung.

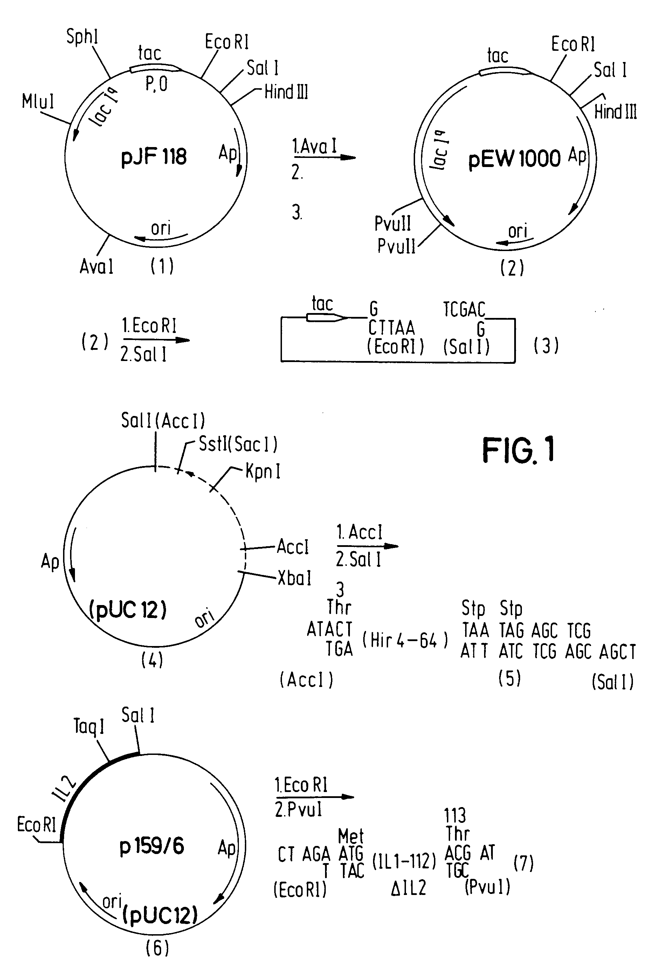

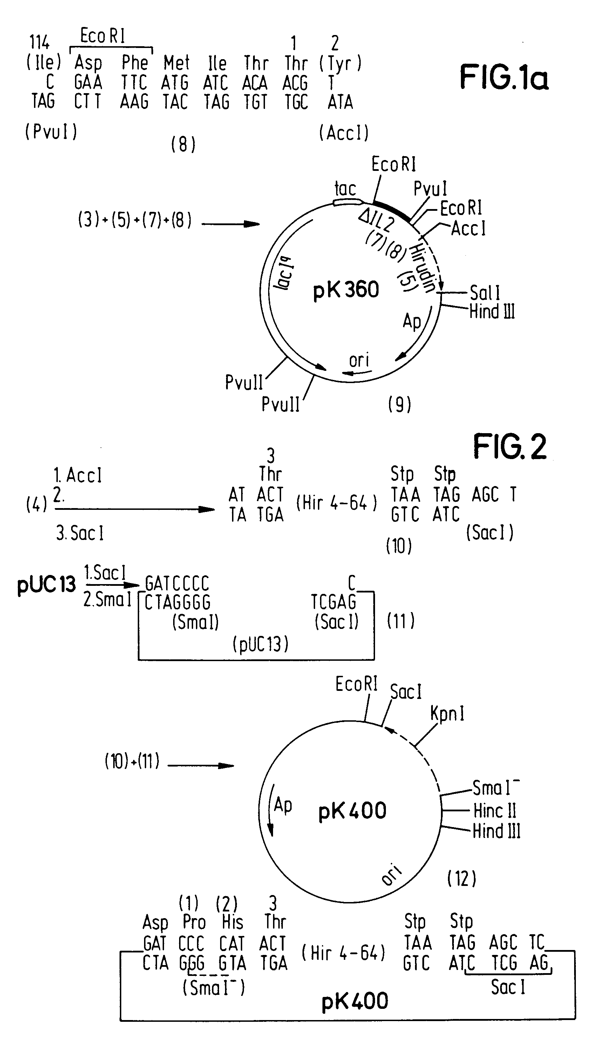

In den folgenden Beispielen und in den Figuren wird die Erfindung näher erläutert. Hierbei beziehen sich Figur 1 und deren Fortsetzung, Figur 1a, auf die Synthese des Plasmids pK360, das für ein Fusionsprotein codiert, welches die Hirudinsequenz aufweist;

Figur 2 und ihre Fortsetzung, Figur 2a, betreffen die Synthese des Plasmids pK410, welches ebenfalls für ein Fusionsprotein mit der Aminosäuresequenz des Hirudin codiert;

Figur 3 auf die Synthese des Plasmids pPH100, welches für ein Fusionsprotein mit der Aminosäuresequenz des Hirudin codiert.

Die Figuren sind i.a. nicht maßstabgerecht gezeichnet, vor allem bei der Wiedergabe der Polylinker wurde der Maßstab "gedehnt".

Beispiel 1

Durch Insertion des lac-Repressors (P.J. Farabaugh, Nature 274 (1978) 765-769) in das Plasmid pKK 177-3 (Amann et al., Gene 25 (1983) 167) erhält man das Plasmid pJF118 (1) (Fig. 1; vgl. deutsche Patentanmeldung P 35 26 995.2, Beispiel 6, Fig. 6). Dieses wird an der singulären Restriktionsstelle für Ava I geöffnet und in an sich bekannter Weise durch Exonuclease-Behandlung um etwa 1000 bp verkleinert. Nach Ligierung wird das Plasmid pEW 1000 (2), (Figur 1) erhalten, in dem das lac-Repressorgen vollständig erhalten ist, das aber auf Grund der Verkleinerung in deutlich höherer Kopienzahl als das Ausgangsplasmid vorliegt.

Anstelle des Plasmids pKK177-3 kann man auch von dem vorstehend erwähnten handelsüblichen Plasmid pKK223-3 ausgehen, den lac-Repressor einbauen und das erhaltene Produkt analog verkürzen.

Das Plasmid pEW 1000 (2) wird mit den Restriktionsenzymen EcoR I und Sal I geöffnet (3).

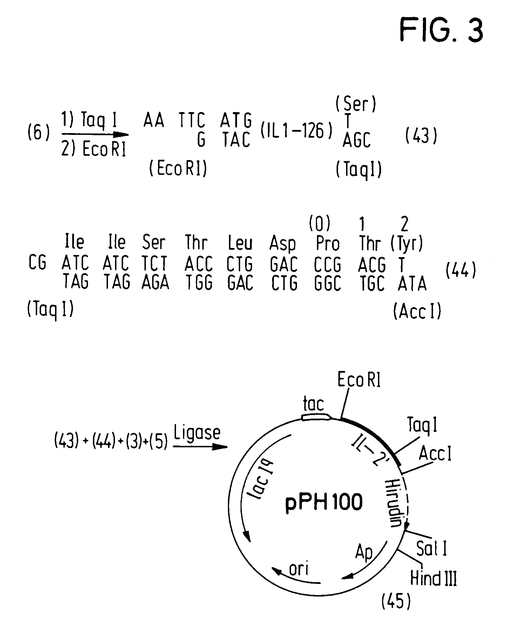

Das für Hirudin codierende Plasmid (4), hergestellt gemäß deutscher Offenlegungsschrift 34 29 430 (europäische Patentanmeldung mit der Veröffentlichungsnummer 0 171 024), Beispiel 4 (Figur 3), wird mit den Restriktionsenzymen Acc I und Sal I geöffnet und das kleine Fragment (5), das zum größten Teil die Hirudin-Sequenz enthält, isoliert.

Das Plasmid p159/6 (6), hergestellt gemäß deutscher Offenlegungsschrift 34 19 995 (europäische Patentanmeldung mit der Veröffentlichungsnummer 0 163 249), Beispiel 4 (Figur 5), wird mit den Restriktionsenzymen Eco RI und Pvu I geöffnet und das kleine Fragment (7) isoliert, das den größten Teil der IL2-Sequenz enthält. Diese Teilsequenz und im folgenden auch andere verkürzte IL-2 Sequenzen sind in den Figuren mit "ΔIL2" bezeichnet.

Anschließend werden die Sequenzen (3), (5), (7) sowie die synthetische DNA-Sequenz (8; Figur 1a) mit T4-Ligase behandelt. Man erhält das Plasmid pK360 (9).

Kompetente E. coli-Zellen werden mit dem Ligationsprodukt transformiert und auf NA-Platten, die 25 μg/ml Ampicillin enthalten, ausplattiert. Die Plasmid-DNA der Klone wird mittels Restriktions- und Sequenzanalyse charakterisiert.

Eine Übernachtkultur aus E. coli-Zellen, die das Plasmid (9) enthalten, wird mit LB-Medium (J. H. Miller, Experiments in Molecular Genetics, Cold Spring Harbor Laboratory, 1972), das 50 μg/ml Ampicillin enthält, im Verhältnis von etwa 1:100 verdünnt und das Wachstum über OD-Messung verfolgt. Bei OD = 0,5 wird die Schüttelkultur auf 1 mM Isopropyl-$\beta$-galactopyranosid (IPTG) eingestellt und die Bakterien nach 150 bis 180 Minuten abzentrifugiert. Die Bakterien werden 5 Minuten in einer Puffermischung (7M Harnstoff, 0,1% SDS, 0,1 M Natriumphosphat, pH 7,0) gekocht und Proben auf eine SDS-Gelelektropheseplatte aufgetragen. Nach Elektrophorese wird aus Bakterien, die das Plasmid (9) enthalten, eine Proteinbande erhalten, die der Größe des erwarteten Fusionsproteins entspricht. Nach Aufschluß der Bakterien (French Press; ®Dyno-Mühle) und Zentrifugation befindet sich das Fusionsprotein im Niederschlag, so daß mit dem Überstand bereits erhebliche Mengen der übrigen Proteine abgetrennt werden können. Nach Isolierung des Fusionsproteins wird durch Bromcyan-Spaltung das erwartete Hirudin-Peptid freigesetzt. Dieses wird nach Isolierung durch Protein-Sequenzanalyse charakterisiert.

Die angegebenen Induktionsbedingungen gelten für Schüttelkulturen; bei größeren Fermentationen sind entsprechend veränderte OD-Werte und gegebenenfalls leicht variierte IPTG-Konzentrationen zweckmäßig.

Beispiel 2

Das Plasmid (4) (Figur 1) wird mit Acc I geöffnet und die überstehenden Enden mit Klenow-Polymerase aufgefüllt. Anschließend wird mit Sac I geschnitten und das Fragment (10) isoliert, das den größten Teil der Hirudin-Sequenz enthält.

Der handelsübliche Vektor pUC 13 wird mit den Restriktionsenzymen Sac I und Sma I geöffnet und das große Fragment (11) isoliert.

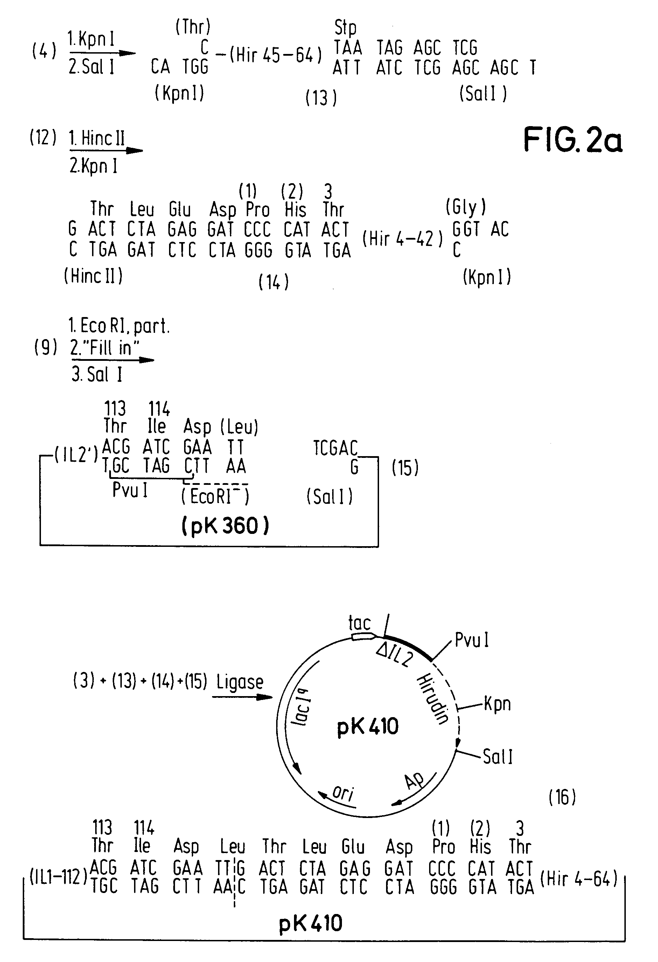

Mittels T4-Ligase werden nun die Fragmente (10) und (11) zum Plasmid pK 400 (12) (Fig. 2) ligiert. Das Plasmid (12) ist in der Figur 2 zweimal dargestellt, wobei in der unteren Darstellung die Aminosäuresequenz des so erhältlichen Hirudin-Derivats hervorgehoben wird.

Das Plasmid (4) (Figur 1) wird mit den Restriktionsenzymen Kpn I und Sal I geöffnet und das kleine Fragment (13) isoliert, das die Hirudin-Teilsequenz enthält.

Das Plasmid (12) wird mit den Restriktionsenzymen Hinc II und Kpn I umgesetzt und das kleine Fragment (14) isoliert, das die Hirudin-Teilsequenz enthält.

Das Plasmid (9) (Figur 1a) wird mit EcoR I partiell gespalten, die freien Enden mit Klenow-Polymerase in einer fill-in-Reaktion aufgefüllt und mit Sal I geschnitten. Man erhält das Derivat des Plasmids pK360 (15).

Durch Ligieren der Fragmente (3), (13), (14) und (15) erhält man das Plasmid pK410 (16), das in der Figur 2a zweifach dargestellt ist, wobei die untere Wiedergabe die Aminosäurefolge des Fusionsproteins und damit des nach Säurespaltung erhaltenen Hirudin-Derivats erkennen läßt.

Nach Expression und Aufarbeitung gemäß Beispiel 1 erhält man ein neues Hirudin-Derivat, das in den Positionen 1 und 2 die Aminosäuren Prolin und Histidin aufweist. Dieses Hirudin-Derivat zeigt die gleiche Aktivität wie das Naturprodukt gemäß deutscher Offenlegungsschrift 34 29 430, das in diesen Positionen die Aminosäuren Threonin und Tyrosin aufweist, ist jedoch stabiler gegen den Angriff von Aminopeptidasen, woraus sich Vorteile bei der in-vivo-Anwendung ergeben können.

Beispiel 3

Das Plasmid (6) (Figur 1) wird mit den Restriktionsenzymen Taq I und Eco RI geöffnet und das kleine Fragment (43) isoliert. Dieses Fragment wird mit der synthetisierten DNA-Sequenz (44) und den Segmenten (3) und (5) zum Plasmid pPH 100 (45) ligiert. Dieses Plasmid codiert für ein Fusionsprotein, bei dem auf die ersten 132 Aminosäuren des IL-2 das Brückenglied Asp-Pro und hierauf die Aminosäurefolge von Hirudin folgt. Die proteolytische Spaltung liefert somit ein modifiziertes, biologisch aktives IL-2', in dem in Position 133 an Stelle von Thr Asp enthalten ist, und ein Hirudin-Derivat, das N-terminal Pro vor der Aminosäuresequenz des Naturprodukts enthält. Auch dieses Produkt ist biologisch aktiv und im Vergleich zu dem Naturprodukt stabiler gegen den Angriff von Proteasen.

**Patentansprüche**
**Patentanspruch für folgende Vertragsstaaten : BE, CH, DE, FR, GB, IT, LI, LU, NL, SE**

**1.** Hirudinderivate, gekennzeichnet durch eine Aminosäurefolge, die N-terminal mit Pro-His oder Pro-Thr beginnt.

**Patentanspruch für folgende Vertragsstaaten : AT, ES, GR**

**1.** Verfahren zur Herstellung von Hirudinderivaten, dadurch gekennzeichnet, daß die Aminosäurefolge des Hirudinderivats mit Pro-His oder Pro-Thr-beginnt.

**Claims**
**Claim for the following Contracting States : BE, CH, DE, FR, GB, IT, LI, LU, NL, SE**

**1.** A hirudin derivative which has an amino-acid sequence starting at the N terminus with Pro-His or Pro-Thr.

**Claim for the following Contracting States : AT, ES, GR**

**1.** A process for preparing hirudin derivatives wherein the amino-acid sequence of the hirudin derivative starts with Pro-His or Pro-Thr.

**Revendications**
**Revendication pour les Etats contractants suivants : BE, CH, DE, FR, GB, IT, LI, LU, NL, SE**

**1.** Dérivés d'hirudine, caractérisés par une séquence d'aminoacides qui commence, à l'extrémité N-terminale, par Pro-His ou Pro-Thr.

**Revendication pour les Etats contractants suivants : AT, ES, GR**

**1.** Procédé pour la production de dérivés d'hirudine, caractérisé en ce que la séquence d'aminoacides du dérivé d'hirudine commence par Pro-His ou Pro-Thr.

FIG. 1

## FIG.1a

```
114    EcoRI                                    1     2
(Ile)  Asp  Phe  Met  Ile  Thr  Thr  (Tyr)
  C    GAA  TTC  ATG  ATC  ACA  ACG    T
 TAG   CTT  AAG  TAC  TAG  TGT  TGC   ATA
(PvuI)                                        (AccI)
                   (8)
```

(3)+(5)+(7)+(8) ⟶

pK360

tac
EcoRI
PvuI
EcoRI
AccI
ΔIL2
(7)(8)
Hirudin
(5)
SalI
HindIII
Ap
lacI^q
ori
PvuII
PvuII
(9)

## FIG.2

```
1. AccI                         3
2.                             Thr           Stp  Stp
(4) ⟶              AT  ACT  (Hir 4-64)  TAA  TAG  AGC  T
3. SacI                     TA  TGA           GTC  ATC
                                                   (10)      (SacI)
```

```
pUC13  1.SacI  ⟶  GATCCCC                      C
       2.SmaI      CTAGGGG            TCGAG
                   (SmaI)            (SacI)        (11)
                        (pUC13)
```

(10)+(11) ⟶

EcoRI  SacI  KpnI
Ap   pK400
SmaI⁻
HincII
HindIII
ori
(12)

```
      (1)  (2)   3
Asp   Pro  His  Thr                  Stp  Stp
GAT   CCC  CAT  ACT  (Hir 4-64)  TAA  TAG  AGC  TC
CTA   GGG  GTA  TGA                  GTC  ATC  TCG  AG
     (SmaI⁻)                                   SacI
                   pK400
```

FIG.2a

(4) 1.Kpn I / 2.Sal I →

(Thr)
C — (Hir 45-64)
CA TGG
(Kpn I)

Stp
TAA TAG AGC TCG
ATT ATC TCG AGC AGC T
(13)                    (Sal I)

(12) 1. Hinc II / 2. Kpn I →

        Thr Leu Glu Asp Pro His Thr           (Gly)
             (1)  (2)  3
      G ACT CTA GAG GAT CCC CAT ACT           GGT AC
      C TGA GAT CTC CTA GGG GTA TGA (Hir 4-42) C
      (Hinc II)              (14)              (Kpn I)

(9) 1. EcoRI, part. / 2."Fill in" / 3. Sal I →

        113  114
        Thr  Ile  Asp (Leu)
-(IL2')ACG ATC GAA  TT          TCGAC
       TGC TAG CTT  AA              G     (15)
       Pvu I   (EcoRI⁻)         (Sal I)
                (pK 360)

(3) + (13) + (14) +(15) Ligase →

pK 410

tac  ΔIL2  Pvu I
lacI⁹  Hirudin  Kpn
pK 410  Sal I
ori  Ap
(16)

        113  114
        Thr  Ile  Asp  Leu  Thr  Leu  Glu  Asp  Pro  His  Thr
                                                    (1)  (2)  3
(IL1-112)ACG ATC GAA TTG ACT CTA GAG GAT CCC CAT ACT (Hir 4-64)
         TGC TAG CTT AAC TGA GAT CTC CTA GGG GTA TGA
                   pK 410

# FIG. 3

$$(6) \xrightarrow[\text{2) EcoRI}]{\text{1) Taq I}} \quad \begin{array}{ccc} \text{AA} & \text{TTC} & \text{ATG} \\ & \text{G} & \text{TAC} \end{array} \text{(IL1-126)} \begin{array}{c} \text{(Ser)} \\ \text{T} \\ \text{AGC} \end{array} \quad (43)$$

(EcoRI)  (TaqI)

$$\begin{array}{ccccccccc} & \text{Ile} & \text{Ile} & \text{Ser} & \text{Thr} & \text{Leu} & \text{Asp} & \overset{(0)}{\text{Pro}} & \overset{1}{\text{Thr}} & \overset{2}{\text{(Tyr)}} \\ \text{CG} & \text{ATC} & \text{ATC} & \text{TCT} & \text{ACC} & \text{CTG} & \text{GAC} & \text{CCG} & \text{ACG} & \text{T} \\ & \text{TAG} & \text{TAG} & \text{AGA} & \text{TGG} & \text{GAC} & \text{CTG} & \text{GGC} & \text{TGC} & \text{ATA} \end{array} \quad (44)$$

(Taq I)  (Acc I)

$$(43) + (44) + (3) + (5) \xrightarrow{\text{Ligase}}$$

pPH 100

EcoRI
tac
TaqI
Acc I
IL-2'
Hirudin
lacIq
Ap
Sal I
Hind III
ori
(45)